## Europäisches Patentamt

# European Patent Office

## Office européen des brevets

(11) Numéro de publication: **0 132 210**
**B1**

# FASCICULE DE BREVET EUROPÉEN

(45) Date de publication du fascicule du brevet: **25.11.87**

(51) Int. Cl.⁴: **A 61 M 1/00,** A 61 M 1/34

(21) Numéro de dépôt: **84420119.4**

(22) Date de dépôt: **05.07.84**

(54) **Appareillage utilisable notamment en plasmaphérèse.**

(30) Priorité: **13.07.83 FR 8311932**

(43) Date de publication de la demande:
**23.01.85 Bulletin 85/4**

(45) Mention de la délivrance du brevet:
**25.11.87 Bulletin 87/48**

(84) Etats contractants désignés:
**AT BE CH DE GB IT LI LU NL SE**

(56) Documents cité:
**DE-A-2 745 041**
**DE-A-3 043 682**
**FR-A-2 195 483**
**US-A-2 766 907**
**US-A-4 191 182**

**Petit Larousse Illustré, p. 199, edition 1986**

(73) Titulaire: **RHONE- POULENC S.A., 25, quai Paul Doumer, F-92408 Courbevoie (FR)**

(72) Inventeur: **Vantard, Georges, 6, impasse Rafo, F-38290 Villefontaine (FR)**

(74) Mandataire: **Vogt, Bernard, RHONE- POULENC INTERSERVICES Service Brevets Chimie Centre de Recherches de Saint- Fons B.P. 62, F-69192 Saint- Fons Cédex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

## Description

La plasmaphérèse est une opération consistant à séparer le sang entier d'un donneur en deux parties, la première partie constituant la phase plasmatique, tandis que la seconde partie constitue la phase cellulaire qui est en général réinjectée au donneur. La phase plasmatique est une solution aqueuse complexe contenant notamment des protéines, tandis que la phase cellulaire contenant encore du plasma comprend les globules rouges (ou hématies), les globules blancs (ou leucocytes) et les plaquettes.

La technique de plasmaphérèse est utilisée depuis fort longtemps en expérimentation animale. A titre indicatif on peut citer l'article de John J. ABEL et al. ayant pour titre "Plasma removal with return of corpuscules", paru dans J. Pharmacol. Exp. Ther. en 1914, n° 5, pages 625 à 641 dans lequel du sang de chien est centrifugé pour faire la séparation. On peut citer également l'article de A. GEIGER paru en 1931 dans J. Phys. 71, pages 111-120 ayant pour titre "Method of Ultrafiltration in vivo" dans lequel il est décrit une opération de plasmaphérèse en continu sur chien, l'appareil de séparation utilisé étant un séparateur à membrane, cette dernière étant disposée en spirale et étant choisie de façon telle qu'il soit possible d'obtenir une solution plasmatique comprenant la totalité des protéines du sang traité, lorsque cela est désiré.

La plasmaphérèse est également appliquée à l'homme depuis un certain nombre d'années comme l'indique l'article "La plasmaphérèse-Technique-Indications" de Fr. OBERLING et al. paru dans J. Med. Strasbourg 1968, mars, page 277-279. La plasmaphérèse tend ainsi maintenant à supplanter le don total du sang car cette technique présente l'avantage de permettre de prélever à l'homme de plus grandes quantités de plasma sans inconvénient. Du fait que l'on restitue au donneur les éléments figurés du sang, les séances de prélèvement peuvent être plus rapprochées dans le temps que pour le don du sang.

Ainsi la plasmaphérèse est une technique ancienanne et les perfectionnements ultérieurs lui ayant été apportés portent soit sur des appareils à centrifugation, soit sur des appareils à membranes. Parmi les brevets de perfectionnement concernant les appareils de centrifugation, on peut citer le DE-A-2 745 041 qui permet de ne piquer le donneur qu'une seule fois et nécessite un récipient de centrifugation particulier. Parmi les brevets de perfectionnement conernant des appareils à membranes, on peut citer DE-A-2 100 209 de AMICON dans lequel il est décrit un récipient comprenant une membrane formant une spirale pour la circulation du sang entier prélevé d'un donneur et dans lequel on exerce une pression sur le sang contenu dans le récipient, soit au moyen d'un gaz, soit au moyen du piston d'une seringue soumis à l'action d'un ressort à lame. Par rapport à l'appareil de GEIGER précédemment décrit, cet appareil de par sa conception présente l'inconvénient de ne pas permettre d'opérer en continu sur le donneur. On peut citer également US-A-4 191 182 de HEMOTHERAPY Inc., dans lequel il est décrit un appareillage à membrane et dans lequel le sang prélevé en continu au donneur est séparé en plasma et en une fraction cellulaire retournant en continu au donneur, cet appareillage ayant notamment pour caractéristique le fait de permettre à une partie de la fraction cellulaire de recirculer dans le compartiment amont de l'appareil à membrane et le fait de permettre à la fraction plasmatique de recirculer dans le compartiment aval du même appareil. On peut citer de plus la demande internationale déposée par FRIEDMAN et al. et publiée sous le n° wo 79/01 121, dans laquelle il est également question d'un appareillage permettant de retirer le sang du donneur et de lui réinjecter la fraction n'ayant pas traversé la membrane, de façon continue. On peut citer en outre le DE-A-3 043 682 concernant un appareil de plasmaphérèse à membrane comportant des capteurs pour contrôler la pression transmembranaire dans celui-ci.

Cependant les appareillages à membranes décrits ci-avant et permettant la plasmaphérèse en continu ont notamment pour inconvénient d'exiger de piquer le donneur en deux endroits distincts, ce qui est assez désagréable pour celui-ci.

Un but de la présente invention est donc un appareillage utilisant un appareil à membrane et permettant des opérations de plasmaphérèse sur un donneur en ne le piquant qu'à un seul endroit avec une aiguille classique de transfusion sanguine.

Un autre but de la présente invention est un appareillage permettant de soumettre le sang en provenance du donneur à une première séparation lors de son passage sur la membrane, puis à une seconde séparation sur la même membrane lors de son retour au donneur, les moyens permettant le retour du sang ne comprenant pas de pompe entre le récipient de recueil du sang ayant passé au contact de la membrane et l'appareil à membrane.

Un autre but de la présente invention est un appareillage spécialement adapté permettant d'obtenir un plasma de très bonne qualité et dans les meilleures conditions de rendement de filtration, tout en assurant une hémolyse pratiquement nulle du sang.

Un autre but de la présente invention est un appareillage permettant de réguler la pression de sortie de la fraction cellulaire du compartiment amont de l'appareil à membrane à des valeurs comprises généralement entre 0 et 20 mm de mercure en pression relative, le compartiment aval étant à la pression atmosphérique. Ainsi un but de la présente invention est un appareillage dans lequel on est sur que la pression transmembranaire du liquide circulant au contact de la membrane ne dépasse pas une valeur de consigne.

Un autre but de la présente invention est un appareillage permettant de retirer d'un donneur environ 600 ml de plasma en environ 45 minutes.

Un autre but de la présente invention est un appareillage de prélèvement de plasma dans lesquels il est possible d'adapter facilement la stratégie opératoire en fonction du donneur, des désirs de l'opérateur et des caractéristiques de l'appareil à membrane utilisée.

Un autre but de la présente invention est un appareillage, dans une variante de réalisation, permettant de faire passer le sang au contact de la menanbrane à un débit supérieur à celui du sang prélevé au donneur, tout en n'ayant pas de pompe entre le récipient de recueil du liquide ayant circulé au contact de la membrane sans la traverser et la sortie du compartiment amont de l'appareil à membrane, lors de la phase de retour. Un but de la présente invention est donc un appareillage permettant d'obtenir un rendement de séance supérieur au rendement intrinsèque de l'appareil à membrane. On entend par rendement de séance le rapport entre le débit de liquide qui a traversé la membrane (c'est-à-dire le plasma dans une opération de plasmaphérèse) et le débit de sang prelevé ou restitué à la veine du donneur. On entend par rendement intrinsèque de l'appereil à membrane le rapport du débit de plasma filtré au débit du sang à l'entrée de l'appareil à membrane.

Un autre but de la présente invention est un appareillage se prêtant également bien à la plasmaphérèse avec restitution au patient, lors de la phase de réinjection (ou de retour), d'un liquide de remplacement dont le volume correspond sensiblement à celui du plasma retiré.

Il a maintenant été trouvé et c'est ce qui fait l'objet de la présente invention, un appereillage utilisable dans le domaine médical, notamment en plasmaphérèse, caractérisé en ce qu'il comprend en combinaison:

- un dispositif (1) de prélèvement d'un liquide d'un sujet,
- un appareil (2) à membrane séparant ce liquide en une fraction ayant traversé la membrane, et en une fraction n'ayant pas traversé la membrane,
- une première canalisation (5) reliant le dispositif (1) de prélèvement à l'entrée (6) du compartiment amont (3) de l'appareil (2) à membrane,
- une pompe (7) disposée sur cette canalisation (5),
- un capteur de pression (14) du liquide circulant dans la canalisation (5), ledit capteur étant situé entre la pompe (7) et l'entrée (6) du compartiment amont (3),
- une deuxième canalisation (16) reliant la sortie (15) du compartiment amont (3) de l'appareil (2) à membrane à un récipient (17) souple pour recueillir la fraction du liquide n'ayant pas traversé la membrane,
- un récipient (20) de recueil du liquide ayant traversé la membrane, ce récipient (20) étant en communication avec la sortie du compartiment aval (4) de l'appareil à membrane (2),
- des moyens pour connaître le volume de liquide prélevé au sujet lors de chaque phase de prélèvement,
- une poche (18) souple gonflable agissant, lorsqu'elle est gonflée, sur le récipient (17) souple et permettant le retour au sujet du liquide contenu dans ce récipient (17) au cours de la phase dite de retour, le dispositif (1) constitué d'une simple aiguille prise de sang servant alors à la réinjection au sujet de cette fraction n'ayant pas traversé la membrane,
- des moyens pour s'assurer que la poche (18) souple n'est pas gonflée au-delà d'une certaine pression de consigne lors de la phase dite de retour,
- des moyens (23, 19) pour gonfler ou dégonfler ladite poche.

La description de la présente invention sera mieux comprise à l'aide des figures ci-jointes, qui illustrent, de façon schématique, à titres d'exemples non limitatifs et sans échelle déterminée, des modes de réalisation particuliers dudit appareillage.

La figure 1 représente un mode de réalisation de l'appareillage selon la présente invention.

La figure 2 représente l'appareillage selon la figure 1 dans lequel ont été notamment ajoutées les connexions à un dispositif de contrôle et de commande.

La figure 3 représente une variante de réalisation de l'appareil selon la figure 1.

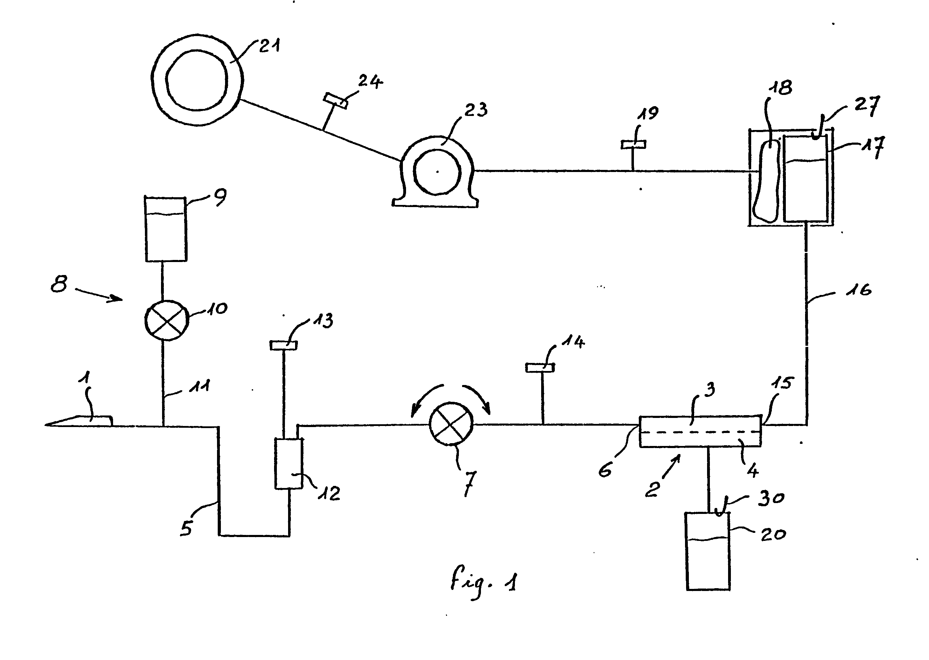

L'appareillage selon la figure 1, plus spécialement adapté à la plasmaphérèse de don, comprend un dispositif (1) de prélèvement du sang du donneur, constitué avantageusement d'une aiguille de prise de sang. A titre indicatif cette aiguille (1) peut avoir un diamètre externe de 1,65 mm et un diamètre interne de 1,45 mm, telle que celles répertoriées dans les centres de transfusion sanguine sous le vocable 16 G. Un appareil (2) à membrane semi-perméable, comportant un compartiment amont (3) et un compartiment aval (4) est relié à l'aiguille (1) par une canalisation (5) allant de l'aiguille (1) à une tubulure (6) en communication avec le compartiment amont (3) de l'appareil (2) à membrane. Cette canalisation (5) est généralement constituée par un tube en matière plastique, par exemple en polychlorure de vinyle. Sur cette canalisation ou ligne (5) se trouve une pompe (7) pouvant tourner dans les deux sens, avantageusement du genre péristaltique. Entre la pompe (7) et l'aiguille (1) est disposé un dispositif (8) destiné à transmettre un produit anticoagulant au sang en provenance du donneur, par exemple une solution glucosée contenant 35,6 g/l de citrate trisodique, référencée AB 16 de la Société BIELUZ. Ce dispositif (8) comprend par exemple un réservoir (9) d'anticoagulant, une canalisation (11) raccordée à la canalisation (5) et au réservoir (9), et une pompe (10) par exemple péristaltique, située sur la canalisation (11). Cette canalisation (11) se raccorde sur la canalisation (5) le plus près

possible de l'aiguille (1). Entre le point de raccordement des canalisations (11 et 5) et la pompe (7), un piège à bulles (12) et un capteur (13) de pression sont avantageusement sur la canalisation (5). La pompe (7) est asservie au détecteur (13) de pression. Un détecteur (14) de pression se trouve entre l'appareil (2) à membrane et la pompe (7), cette dernière étant asservie à ce détecteur (14). La tubulure (15) du compartiment amont (3) de l'appareil (2) à membrane est reliée par un canalisation (16) à un récipient (17) souple de recueil du sang ayant passé au contact de la membrame sans la traverser, ce récipient (17) étant par exemple une poche en matière plastique souple. Cette canalisation (16) peut être en même matière et aux mêmes diamètres que la canalisation (5). Le compartiment aval (4) de l'appareil (2) est relié à un récipient (20) de recueil du plasma ayant traversé la membrane, ce récipient (20) étant par exemple (comme le récipient 17) une poche fermée en matière plastique souple dont les deux parois opposées sont en contact l'une avec l'autre lorsque la poche est vide, et dont les deux parois s'écartent l'une de l'autre lorsqu'un liquide pénètre à l'intérieur sous une très légère pression. De telles poches (17 ou 20) sont commercialisées par la Société FENWAL sous la référence "transfer-pack" R 2022. L'appareillage comporte des moyens, par exemples des pesons électroniques (27 et 30) pour connaître le contenu des récipients (17 et 20).

L'appareillage selon la figure 1 comprend de plus un garrot (21) connu en soi et des moyens, par exemple notament un compresseur (23) poui le gonfler à la pression souhaitée lorsque cela est voulu, un capteur de pression arrêtant le gonflement du garrot (21) lorsqu'une pression de consigne est atteinte. L'appareil selon la figure 1 comprend une poche gonflable (18) qui peut être gonflée par le compresseur (23) à la pression souhaitée grâce au capteur de pression (19), ladite poche gonflable agissant et transmertant sa pression au récipient (17) qui lui est contigu, grâce par exemple à une enveloppe rigide transparente dans laquelle sont contenus à la fois la poche (18) et le récipient souple (17).

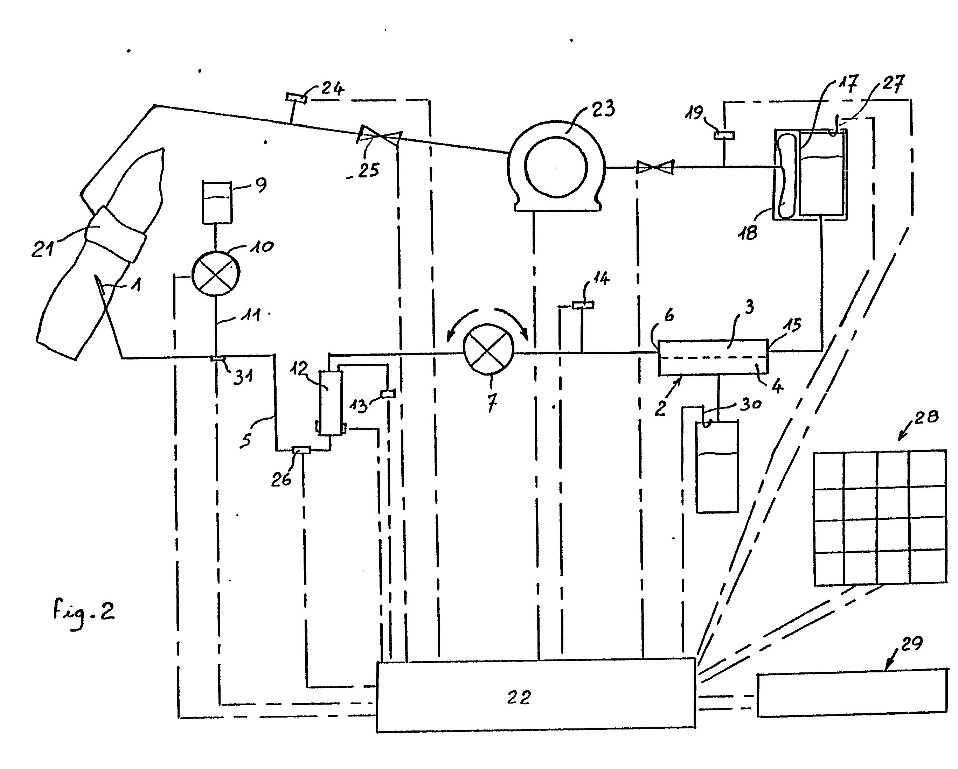

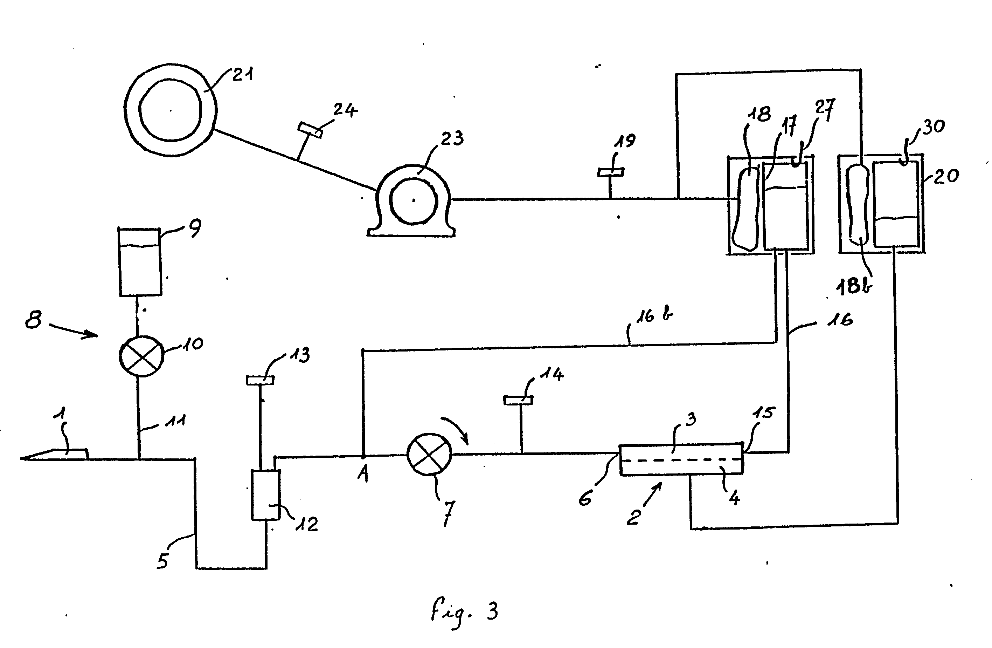

La figure 2 représente un appareillage équivalent à celui selon la figure 1, mais dans lequel on trouve les connexions électriques des différents éléments à une unité logique (22) de commande et de contrôle, les lignes électriques étant représentées en traits mixtes. L'unité (22) est reliée à une source de courant (non représentée). Biem entendu tous les éléments de l'appareillage peuvent être groupés sur une console (ou pupitre) comprenant par exemple des roulettes, pour mieux la déplacer. La description de l'unité logique (22) ainsi que du clavier (28) et de l'afficheur (29) dont il sera question ci-après n'a pas été développée car la réalisation des circuits électriques et l'utilisation avantageuse de micro-processeurs et de mémoires est évidente pour le technicien considéré une fois le problème posé, c'est-à-dire après qu'on lui ait demandé de faire en sorte que l'appareillage décrit puisse fonctionner de façon automatique et fiable. L'appareillage, dont il sera question à la figure 3, est avantageusement, lui aussi, relié à une unité (22) logique de commande et de contrôle non représentée.

L'appareil (2) dont il a été question ci-avant peut avoir sa membrane sous forme plane, sous forme spiralée, ou sous forme de petits tubes fins tels que des fibres creuses. Lorsque la membrane comprend une pluralité de fibres creuses, le sang circule avantageusement à l'intérieur des fibres creuses, l'ensemble des parties internes des fibres constituant le compartiment (3) amont de l'appareil. Lorsque la membrane est sous forme plane ou spiralée, le sang circule avantageusement entre deux membranes ou des groupes de deux membranes qui constituent le compartiment amont (3) de l'appareil (2) à membrane.

Les membranes utilisées pour des opérations de plasmaphérèse sont de préférence celles qui pemettent de recueillir un plasma: - dans lequel toutes les protéines du sang initial sont retrouvées dans les mêmes proportions, - dont la concentration protéique est supérieure à 55,5 g/l, - dans lequel il n'y a pas de globules rouges et dans lequel la concentration plaquettaire est inférieure à 15 000 plaquettes par $mm^3$. Les membranes choisies sont celles permettant également de ne pas hémolyser le sang circulant à leur contact, tout en offrant de bons rendements de filtration.

Ces membranes de plasmaphérèse ont avantageusement un taux de rejet au latex, dont les particules sont calibrées à 0,27 microns, inférieur à 75 % et elles ont un taux de rejet au latex, dont les particules sont calibrées à 0,64 microns, supérieur à 15 %.

Préférentiellement le taux de rejet aux particules calibrées à 0,27 microns est inférieur à 30 % et le taux de rejet aux particules de latex calibrées à 0,64 microns est supérieur à 90 %.

Pour effectuer cette mesure de taux de rejet au latex, on opère de la façon suivante, lorsque les membranes sont planes.

Dans une cellule de type AMICON Modèle 52, on charge 50 ml de suspension de particules calibrées de polystyrène de diamètre 0,27-0,4 ou 0,64 microns (vendues par la Société RHONE-POULENC sous la marque ESTAPOR) diluée à 0,1 % avec de l'eau distillée additionnée de 1 % d'agent tensio-actif (alkylarylsulfonate marque SINOZON NAS 60, fabriqué par la Société SINNOVA).

La cellule AMICON est équipée d'un échantillon de la membrane tramée. On établit une pression d'air correspondant à 20 cm d'eau. On récupère les six premiers millilitres de filtrat dont on détermine la concentration (cf) en particules calibrées.

Le taux de rejet est défini par la formule:

$$\frac{(0,1 - cf) \times 100}{0,1}$$

Des membranes ayant les caractéristiques ci-avant sont généralement en matériau synthétique, par exemple en esters de cellulose nitrate de cellulose ...), en cellulose régénérée, en polycarbonate ... Ces membranes peuvent être également à base de polyétheruréthanes comprenant des groupements ammonium héparinés, ou en copolymère d'acrylonitrile. De façon avantageuse ces membranes sont renforcées par une trame lorsqu'elles sont sous forme de membranes planes et ont une épaisseur comprise entre 50 et 200 microns.

Pour la mise en oeuvre de l'appareillage selon les figures 1 et 2 on opère de la façon suivante, par exemple dans le cas d'une opération de plasmaphérèse de don. On commence par remplir la canalisation (11) de solution citratée et comme la jonction de la canalisation (11) avec la canalisation (5) est en fait très proche de l'aiguille (1), on peut considérer que l'aiguille (1) est au moins en partie remplie de cette solution citratée. Le garrot ayant été préalablement gonflé à la pression souhaitée (aux environs de 60 mm de mercure) grâce au compresseur (23), au détecteur de pression (24) et à l'électrovanne (25), [ces deux derniers éléments étant connectés à l'unité logique 22], on enfonce l'aiguille (1) dans une veine du donneur après avoir préparé classiquement l'endroit de piqure, situé entre le garrot et l'extrémité du membre. A ce moment la pompe (10) envoie du citrate dans la canalisation (5), tandis que la pompe (7) tourne et mène le sang du donneur jusqu'au récipient (17) en le faisant passer par le compartiment (3) amont de l'appareil (2) à membrane où une partie du plasma traverse la membrane pour aller dans le compartiment aval (4) relié à la poche (20). Le capteur (13) de pression asservit la pompe (7) de façon à ce que la pression mesurée à cet endroit de la ligne (5) reste toujours supérieure à une certaine valeur, généralement proche de 0 mm de mercure, appelée pression de seuil, pour s'assurer que la pompe (7) ne "tire" pas le sang directement de la veine du donneur. Si la pression dans cette partie de la ligne devient inférieure à la consigne fixée au capteur (13), automatiquement l'unité logique (22) agit et arrête ou ralentit momentanément la rotation de la pompe (7), tant que la pression voulue n'est pas revenue. Le capteur de pression (14) est réglé de façon à ce qu'une pression à l'entrée (6) du compartiment amont (3) supérieure à une valeur maximale, comprise par exemple entre 40 et 100 mm de mercure en valeur relative, de préférence entre 60 et 90 mm de mercure, soit automatiquement détectée. La pompe (7) tourne de façon à ce que la pression du sang à l'entrée du compartiment amont (3) de l'appareil (2) soit le plus près possible de la pression maximale souhaitée, mais que si cette valeur maximale souhaitée est dépassée, automatiquement l'unité logique (22) arrête la pompe (7). La pression à la sortie (15) du compartiment amont (3) du séparateur (2) à membrane est comprise entre 0 et 20 mm de mercure en valeur relative, tandis que le compartiment aval (4) est à la pression atmosphérique.

La période pendant laquelle le sang sort de la veine du donneur est appelée phase de prélèvement. Celle-ci se termine par exemple en fonction du volume prédéterminé de sang que l'on veut prélever au donneur, ce volume étant bien entendu toujours inférieur au volume du récipient souple (17) de recueil du sang. De façon avantageuse un dispositif tachymétrique est branché sur la pompe (7) et lorsque le volume souhaité de sang est prélevé au donneur, l'unité logique (22) agit et arrête la pompe (7). L'unité logique (22) agit alors simultanément sur l'électrovanne (25) qui se positionne de façon telle que le garrot (21) se dégonfle, elle met la pompe (7) en rotation dans le sens inverse de celui de la phase précédente, dite de prélèvement, arrête la distribution du produit anticoagulant contenu dans (9) et met la poche gonflable (18) en pression grâce au compresseur (23), au capteur de pression (19) et à une électrovanne non représentée. La pression de la poche gonflable (18), et ainsi du sang contenu dans le récipient souple (17), est la même que celle précédemment utilisée, pendant la phase de prélèvement, à l'entrée du compartiment amont (3), c'est-à-dire comprise entre 40 et 100 mm de mercure en valeur relative. Au cours de cette phase de retour, pendant laquelle la pompe (1) de distribution de produit anticoagulant est arrêtée, le sang du récipient (17) souple subit une nouvelle séparation dans l'appareil (2) à membrane où il entre dans le compartiment (3) amont par la tubulure (15), et du plasma est recueilli dans le récipient (20). Au cours de cette phase de retour le capteur (13) permet de s'assurer que le sang retournant au donneur ne dépasse pas une certaine valeur maximale de consigne tout en s'y tenant le plus près possible. Si cette valeur maximale de pression est dépassée, par exemple par obturation de l'aiguille, le capteur (13) agit sur la pompe (7) qui lui est asservie et l'unité logique arrête la pompe (7). Le capteur (14) permet de s'assurer que la pression de sortie du compartiment amont (3) est à la pression souhaitée, c'est-à-dire voisine de zéro mm de mercure. Si cette pression est supérieure à la pression de consigne souhaitée la pompe (7) qui est asservie à ce capteur (14), est accélérée dans sa rotation, dans la mesure où la pression maximale du capteur (13) n'est pas atteinte, sinon ce capteur (14) permet d'agir sur la pression de la poche (18) gonflable en la faisant se dégonfler un peu. Si au cours de son retour au donneur des bulles sont décelées par le détecteur (12) l'unité logique (22) arrête immédiatement la pompe (7) et éventuellement agit sur un "clamp" (26), ou dispositif d'obturation, qui ferme la canalisation (5). Le sang peut éventuellement passer au travers d'un filtre classique prévu dans le détecteur à bulles (12) pour éviter que des particules indésirables puissent être renvoyées au donneur. Ce filtre peut par exemple s'effacer lors de la phase de prélèvement, tandis qu'il se rabat sur un siege, prévu dans le détecteur de bulles, lors de la phase de retour. La fin de la phase de retour est détectée, par exemple par un détecteur optique (27) prévu sur la ligne (5). Lorsqu'il ne passe plus de sang (privé d'une partie de son plasma) à l'endroit où se trouve le détecteur (27), l'unité logique (22) intervient pour arrêter la phase de retour et faire repasser l'appareillage sur une phase de prélèvement. Ainsi la pompe (7) est arrêtée et mise en mouvement en tournant, en sens inverse à celui de la phase de retour, tandis que le garrot (21) est gonflé, la pompe (10) de citrate mise en route et la poche (18) souple est dégonflée. Lorsqu'en fin d'une phase de retour on s'aperçoit que la poche (20) à plasma contient une quantité suffisante de plasma, on arrête complètement l'opération.

Généralement le débit de la pompe (10) est réglé de façon que, lors de la phase dite de prélèvement, on ait un volume de citrate pour 8 volumes de sang ou de préférence 1 volume de citrate pour 16 volumes de sang, le rapport étant choisi par l'utilisateur. Ce rapport de dilution est avantageusement obtenu en asservissant la vitesse de rotation de la pompe (10) a celle de la pompe (7).

Il apparaît clairement que l'appareillage selon la présente invention peut faire l'objet d'une automatisation très poussé. Ainsi comme cela est réprésenté plus particulièrement sur la figure 2, l'unité logique (22) de commande et de contrôle peut être reliée à un clavier (28) et à un afficheur (29). De même l'unité logique (22) peut être reliée à un tableau synoptique (non représenté) sur lequel la localisation de toute anomalie est signalée par un voyant lumineux à l'opérateur, en même temps par exemple qu'un signal sonore est mis en marche. Sur le clavier (28) à touches on peut choisir le volume maximum de sans que l'on veut faire circuler pendant la phase de prélèvement (300, 350, 400, 450 cm³ de sang par exemple) en appuyant sur la touche correspondante. On peut également choisir le volume de plasma que l'on veut prélever durant la séance (400, 500 ou 600 cm³ par exemple) en appuyant sur la touche correspondante. Ainsi un dispositif (30), du genre peson électronique par exemple, est avantageusement prévu sur la poche (20) de plasma pour connaître instantanément le volume (ou le poids) de plasma prélevé en cours de séance, ce dispositif (30) comnu en soi étant connecté à l'unité logique (22). Sur le clavier (28) peut également être prévue une touche pour l'amorçage automatique de la ligne (11) avant de piquer le donneur. En appuyant sur cette touche la pompe (10) se met en route et s'arrête automatiquement lorsque la solution de citrate est décelée par exemple à la jonction (31) des deux lignes (11 et 5). Sur le clavier (28) on peut également prévoir par exemple une touche pour connaître sur l'afficheur (29) le volume instantané de plasma dans la poche (20) à tout moment, une touche pour connaître sur l'afficheur (29) le débit du sang de la pompe (7), pour connaître le temps de séance en cours... L'appareillage peut comprendre, en liaison avec l'unité logique (22) et les valeurs affichées au clavier (28) en ce qui concerne le volume de sang souhaité pendant la phase de prélèvement et le volume total de plasma désiré, un système intégrateur agissant au cours de la dernière phase de prélèvement pour que le volume de prélèvement permette d'obtenir le volume total souhaité de plasma à la fin de la dernière phase de retour.

De nombreuses variantes de l'appareillage précédement décrit apparaîtront au technicien. A titre d'exemple l'appareillage peut comporter un ballonnet collabable sur la ligne (5) entre la jonction (31) et le clamp (26). Ce ballonnet sert alors de double sécurité avec le capteur de pression (13) en ce sens qu'il se bouche lorsque le débit de la pompe (7) est supérieur à celui de la veine, si le capteur n'a pas fonctionné au cours de la phase de prélèvement. Eventuellement ce ballonnet collabable peut être substitué au capteur (13).

De même le dispositif (8) destiné à transmettre le produit anticoagulant peut éventuellement être omis dans la mesure où l'intérieur de l'aiguille (1), du détecteur de bulles (12) et des lignes (5 et 16) est par exemple recouvert d'un polymère a base de polyétheruréthanes à groupes ammonium héparinés, tels ceux décrits notamment dans le brevet américain 4 046 725. Eventuellement les lignes (5 et 16) peuvent être en un polymère tel que ceux décrits dans le brevet américain cité ci-avant ou en un mélange de polychlorure de vinyle et de polyétheruréthane à groupes ammonium héparinés tel que ceux des mélanges dont il est question dans la demande de brevet européen n° 12 701. La membrane microporeuse peut elle aussi être préparée à partir d'un mélange de polymères selon la demande de brevet européen n° 12 701.

Avec l'appareillage tel que représenté figures 1 et 2 et précédemment décrit, des opérations de plasmaphérèse ont été effectuées sur un donneur en utilisant à titre d'exemple un appareil (2) à membrane dont la surface membranaire totale est de 600 cm² et comprend deux membrames disposées face à face (constituant le compartiment amont 3) entre lesquelles le sang circule. Les membranes ont chacun 30 cm de longueur et 10 cm de largeur et sont tramées, comme ceci est mieux décrit ci-après. L'épaisseur moyenne du film sang est de 370 microns. Le dispositif (1) de prélèvement est une aiguille de 1,65 mm de diamètre externe et de 1,45 mm de diamètre intérieur. Les canalisations (5 et 6) sont en polychlorure de vinyle (PVC) et ont un diamètre intérieur de 3,5 mm. La canalisation (11) est en PVC et a un diamètre interne de 0,9 mm. La pompe (10) est une pompe péristaltique (référence RP 04 commercialisée par la Société HOSPAL), ladite pompe ayant un corps de pompe en silicone.

La pompe (7) est une pompe péristaltique (référence RP 01, commercialisée par la Société HOSPAL), ladite pompe ayant un corps de pompe en silicone. Les récipients (17 et 18) ont une contenance de 1 000 cm³ et sont en PVC.

Le capteur (13) est un capteur de marque NATIONAL SEMICON DUCTOR, référence LX 1801 GB, dont la pression affichée est réglée à 10 mm de mercure lors de la phase de prélèvement et la valeur maximale de pression est réglée à 100 mm de mercure lors de la phase de retour. Les capteurs (14 et 19) sont des capteurs de même marque et de même référence que le capteur (13). Le capteur (14) est réglé à une pression relative maximale de 80 mm de mercure pour la phase de prélèvement et pour une pression relative minimale de 10 mm de mercure lors de la phase de retour, tandis que le capteur (19) est réglé à une pression relative de 80 mm de mercure pour la phase de retour. Ainsi la pression du sang en circulation est supérieure à la pression du plasma recueilli dans le compartiment (4) aval de l'appareil à membrane qui est à la pression atmosphérique. La pression transmembranaire moyenne est égale à $\frac{80+10}{2}=45$ mm de mercure.

Durant chaque phase de prélèvement le garrot est gonflé à 60 mm de mercure et le débit du sang citraté à l'entrée du séparateur est de 85 ml/mm en moyenne.

La membrane utilisée est une membrane tramée obtenue à partir d'une solution dans un solvant organique de polymère que l'on coule sur une trame tournant au contact d'une bande dont la surface est très lisse. Cette solution comprend 8 % en poids dans un mélange N-méthylpyrolidone/glycérine (70,8/21,2 %) d'un copolymère d'acrylonitrile/méthacrylate de méthyle/méthallyle sulfonate de sodium, comprenant 7,75 % en poids de méthacrylate de méthyle et 80 mEg/kg de sites acides. Ce copolymère a une viscosité spécifique de 0,3 à 20°C en solution à 2 g/l dans le diméthylformamide.

La trame utilisée est un tissu monofilament en polytéréphtalate d'éthylène-glycol, dont la maille est de 75 microns, le diamètre du fil est de 55 microns et le taux de vide est de 33 %. Le grammage de cette trame est de 55 g/m².

La membrane tramée microporeuse obtenue a une épaisseur de 120 microns et son grammage est de 10 g de polymère par m² de membrane sèche.

La microstructure de la phase polymère de la membrane est spongieuse et régulière. Sa porosité est de 80 %, la porosité étant définie comme le rapport (multiplié par 100) entre le volume des pores sur le volume total de la membrane (polymère + pores).

Le débit à l'eau (additionnée de 1 % d'un agent tensio-actif) de cette membrane tramée est de 4,5 ml/h cm² mmHg.

Le taux de rejet au latex de cette membrane est de:
- 5 à 15 % pour un latex calibré à 0,27 microns,
- 65 a 80 % pour un latex calibré à 0,4 microns,
- 98 à 100 % pour un latex calibré à 0,64 microns.

Avec l'appareillage tel que précédemment défini, en se fixant un volume sang de 350 cm³ lors de la phase de prélèvement, un volume total de 600 cm³ de plasma à recueillir et un rapport volume solution anticoagulant/volume sang de 1/16, pendant chaque phase de prélèvement, l'opération de plasmaphérèse a été terminée en 44 minutes après avoir effectué 6 phases de prélèvement et 6 phases de retour.

Le plasma recueilli est pratiquement acellulaire. Il ne contient aucune contamination en globules rouges et seulement 3 000 plaquettes par mm³. La concentration protéïque du plasma est de 57 g/l.

L'appareillage selon la figure 3 est une variante de l'appareillage selon la figure 1, dans lequel nous retrouvons les éléments précédemment décrits avec en plus une canalisation (16b) reliant le récipient (17) souple à la ligne (5), généralement en un point (A) situé entre la pompe (7) et le piège (12) à bulles. Cet appareillage comprend de plus une seconde poche gonflable (18b) agissant sur le récipient (20) souple pour transmettre la pression souhaitée au liquide contenu dans ce récipient (20) lors de chaque phase de retour, cette pression étant avantageusement la même dans les poches (18 et 18b). La présence du capteur (13) est cependant facultative dans cet appareillage selon la figure 3.

L'appareillage selon la figure 3 présente l'avantage par rapport à l'appareillage selon la figure 1 précédemment décrit, de permettre une recirculation du sang, à une vitesse supérieure à sa vitesse de prélèvement ou de retour, dans la boucle définie par le compartiment amont (3) de l'appareil à membrane, la canalisation (16), le récipient souple (17), la canalisation (16b), la portion de la canalisation (5) comprise entre le point A et la tubulure (6) de l'appareil (2) à membrane. Ainsi le rendement d'une séance, par exemple de plasmaphérèse, est supérieur au rendement intrinsèque de l'appareil.

Pour la mise en oeuvre de l'appareillage selon la figure 3 on opère par exemple de la façon suivante dans le cas d'une séance de plasmaphérèse de don. On commence par brancher l'aiguille (1) dans une veine du donneur selon le même protocole que celui décrit pour l'appareillage selon la figure 1, après avoir gonflé le garrot (21) à la pression souhaitée (par exemple 60 mm de mercure), mais on ne met pas en marche la pompe (7) tant qu'un volume Vo de sang n'est pas atteint dans le récipient souple (17). Ainsi le sang passe d'abord par la canalisation (5) jusqu'à la jonction A (point A) puis par la canalisation (16b), la pompe (10) du dispositif anticoagulant fonctionnant comme décrit pour l'appareillage selon la figure 1. Lorsque ce volume (Vo) est atteint on met en marche la pompe (7) de façon à ce que le sang entre dans le compartiment amont (3) de l'appareil à membrane (2) par la tubulure (6). Le capteur (14) de pression permet de choisir la pression transmembranaire la plus forte possible sans qu'il y ait hémolyse, la pression transmembranaire à la sortie (15) du compartiment amont (3) étant sensiblement égale à 0 mm de mercure. Généralement la pression transmembranaire à l'entrée de l'appareil (2) à membrane est comprise entre 40 et 100 mm de mercure. Si la pression fixée est dépassée, le capteur (14) agit sur la pompe (7) qui lui est asservie pour la ralentir ou l'arrêter momentanémment. Au cours de cette phase de prélèvement les récipients souples (17) de recueil du sang ayant passé au contact de la membrane sans la traverser et (20) de recueil du plasma sont sensiblement à la pression atmosphérique. Au cours de cette phase de prélèvement le sang venant du donneur est prélevé comme lors d'une prise de sang classique, puisque la pompe (7) ne l'aspire pas directement mais agit plutôt sur le sang du récipient (17). Lorsque les quantités prédéterminées souhaitées de sang et de plasma sont recueillies dans les poches (17) et (20), ceci étant constaté par exemple grâce aux pesons électroniques (27 et 30), on arrête alors la phase de prélèvement, pour commencer une phase de retour. Le garrot (21) est alors dégonflé, la pompe (10) est arrêtée, les poches gonflables (18 et 18b) sont gonflées toutes deux à une pression correspondant à celle de la pression d'entrée du sang dans l'appareil (2) à membrane lors de la phase de prélèvement, la pompe (7) continue à tourner dans le même sens et la pression de consigne au capteur (14) est augmentée par rapport à celle de la phase de prélèvement de la pression des poches (18 et 18b) de manière à ce que la pression transmembranaire soit la même lors de la phase de retour que celle lors de la phase de prélèvement. Lorsque le volume (Vo) est atteint dans le récipient (17) on recommence alors une phase de prélèvement comme décrite ci-avant en remettant le garrot sous pression, dégonflant les poches (18 et 18b) mettant en marche la pompe (10) et abaissant la valeur de consigne de la pression d'entrée du sang dans le compartiment amont (3) de l'appareil (2) à membrane, la pompe (7) tournant toujours dans le même sens. La fin de cette deuxième phase de prélèvement sera atteinte lorsque le volume extracorporel correspondra par exemple au volume du sang dans le récipient (17) plus le volume du plasma dans le récipient (20), moins le volume de plasma mis en mémoire recueilli en (20) lors de la première phase de prélèvement. Il est à noter que lors de chaque phase de prélèvement dans l'appareillage selon la figure 3, la pompe (10) de distribution de produit anticoagulant n'est plus asservie à la pompe (7) mais avantageusement au volume de sang recueilli dans le récipient (17).

L'appareillage tel que précédemment décrit peut bien évidemment être utilisé sur les animaux (chien, cheval ...) notamment pour des opérations de plasmaphérèse.

De façon générale, cet appareillage peut être utilisé chaque fois qu'il est souhaité de ne piquer un sujet (homme ou animal) qu'à un seul endroit, avec une simple aiguille (n'ayant qu'un canal interne), en faisant circuler le liquide prélevé sur le sujet, généralement du sang d'abord dans un sens (phase de prélèvement), puis dans le sens contraire (phase de retour) au contact d'une membrane d'un appareil à membrane, en ayant des moyens pour maîtriser les pressions désirées d'entrée et de sortie du sang de l'appareil à membrane, lors des deux phases. Ainsi avec cet appareillage il est possible d'éliminer des éléments du sang en circulation, une première fois lors de la phase de prélèvement, et une deuxième fois lors de la phase de retour pendant laquelle la fraction du sang n'ayant pas passé à travers la membrane est restituée au sujet.

Ainsi l'appareillage tel que précédemment décrit peut être utilisé pour d'autres applications que la plamaphérèse de don. En fonction des caractéristiques de séparation des membranes semi-perméables utilisées, il sera possible de n'appauvrir le sang en circulation que de certaines de ses protéines ou que de certains éléments constitutifs du plasma. On peut aussi procéder à des séances d'hémofiltration moyennant par exemple la réinjection d'un liquide de substitution asservie à la quantité de liquide filtré recueilli dans le récipient (20).

Cet appareillage peut être également utilisé pour des opérations d'échange plasmatique, c'est-à-dire en réinjectant au patient, lors de la phase de retour, un plasma em quantité équivalente à celle soutirée, grâce à une pompe et une canalisation (non représentées) se raccordant par exemple sur la canalisation (5) entre le détecteur (12) de bulles et la pompe (7). Si le patient a un shunt artério-veineux il n'est pas nécessaire d'utiliser le garrot (21).

L'appareillage selon la présente invention peut également être utilisé pour le traitement du liquide d'ascite d'un patient, le garrot n'étant pas nécessaire dans cette application, de même que le dispositif (8) de distribution du produit anticoagulant et que le détecteur de bulles (12).

**Revendications**

1. Appareillage utilisable dans le domaine médical, caractérisé en ce qu'il comprend:
   - un dispositif (1) de prélèvement d'un liquide d'un sujet,
   - un appareil (2) à membrane séparant ce liquide en une fraction ayant traversé la membrane, et en une fraction n'ayant pas traversé la membrane,
   - une première canalisation (5) reliant le dispositif (1) de prélèvement à l'entrée (6) du compartiment amont (3) de l'appareil (2) à membrane,
   - une pompe (7) disposée sur cette canalisation (5),
   - un capteur de pression (14) du liquide circulant dans la canalisation (5), ledit capteur étant situé entre la pompe (7) et l'entrée (6) du compartiment amont (3),

- une deuxième canalisation (16) reliant la sortie (15) du compartiment amont (3) de l'appareil (2) à membrane à un récipient (17) souple pour recueillir la fraction du liquide n'ayant pas traversé la membrane,

- un récipient (20) de recueil du liquide ayant traversé la membrane, ce récipient (20) étant en communication avec la sortie du compartiment aval (4) de l'appareil à membrane (2),

- des moyens pour connaître le volume de liquide prélevé au sujet lors de chaque phase de prélèvement,

- une poche (18) souple gonflable agissant, lorsqu'elle est gonflée, sur le récipient (17) souple et permettant le retour au sujet du liquide contenu dans ce récipient (17) au cours de la phase dite de retour, le dispositif (1) constitué d'une simple aiguille de prise de sang servant alors à la réinjection au sujet de cette fraction n'ayant pas traversé la membrane,

- des moyens pour s'assurer que la poche (18) souple n'est pas gonflée au-delà d'une certaine pression de consigne lors de la phase dite de retour,

- des moyens (23, 19) pour gonfler ou dégonfler ladite poche.

2. Appareillage selon la revendication 1, caractérisé en ce qu'il comprend de plus:

- un garrot gonflable (21),

- des moyens (23, 24, 25) permettant de gonfler le garrot (21) lors de la phase de prélèvement et de le dégonfler lors de la phase de retour.

3. Appareillage selon l'une quelconque des revendications précédentes, caractérisé en ce qu'il comprend une dispositif (8) pour introduire, lors de la phase de prélèvement, un anticoagulant dans la canalisation (5), à proximité du dispositif (1) de prélèvement.

4. Appareillage selon l'une quelconque des revendications précédentes, caractérisé en ce qu'il comprend de plus des moyens (30) pour connaître à tout moment la quantié de liquide contenue dans le récipient (20) et des moyens pour passer d'une phase de prélèvement à une phase de retour et vice-versa jusqu'à obtenir le volume souhaité de liquide dans le récipient (20), de préférence en fin d'une phase de retour.

5. Appareillage selon l'une quelconque des revendications précédentes, caractérisé en ce qu'il comprend de plus un détecteur (12) de bulles et un capteur (13) de pression entre le dispositif de prélèvement (1) et la pompe (7).

6. Appareillage selon l'une quelconque des revendications 4 à 5, caractérisé en ce qu'au cours de chaque phase de prélèvement le capteur (13) de pression agissant sur la pompe (7) qui lui est asservie permet de s'assurer que la pression du liquide dans la portion de la ligne (5) située entre le dispositif de prélèvement (1) et ladite pompe (7) ne descend pas en deça d'une pression relative minimale prédéterminée, appelée pression de seuil, caractérisé en ce que le capteur (14) agissant sur la pompe (7) qui lui est asservie permet de s'assurer que la pression d'entrée du liquide entrant dans le compartiment amont (3) de l'appareil (2) à membrane est le plus proche possible d'une pression maximale de consigne sans la dépasser, tandis que le récipient souple (17) et le récipient (20) sont sensiblement à la pression atmosphérique, caractérisé en ce qu'à chaque phase de retour le liquide contenu dans le récipient souple (17) est mis en pression, à une pression prédéterminée, par gonflement de la poche (18) tandis que la pompe (7) tourne en sens inverse, pour que ce liquide retourne au dispositif (1) de prélèvement en passant par la canalisation (16) dans le compartiment amont (3) de l'appareil à membrane et entre dans ledit compartiment à la pression prédéterminée voulue, la pression du liquide dans le récipient (20) étant à la pression atmosphérique, caractérisé en ce que le capteur de pression (13) agit sur la pompe (7) qui lui est asservie pour que ce liquide retournant au sujet n'ait pas une pression supérieure à une valeur de consigné déterminée et qu'il se tienne le plus proche possible de cette valeur de pression, en ce que le capteur de pression (14) agit sur la pompe (7) qui lui est asservie pour que la pression à la sortie de l'appareil (2) à membrane soit le plus près possible de la pression atmosphérique, le capteur (14) agissant éventuellement pour abaisser la pression de la poche gonflable (18) et ainsi du liquide contenu dans le récipienant (17).

7. Appareillage selon l'une quelconque des revenaidications 1 à 5, caractérisé en ce qu'il comprend de plus une canalisation (16b) allant du récipient souple (17) à la ligne (5) avec laquelle elle est raccordée, de preférence entre le piège à bulles (12) et la pompe (7), en ce que le récipient (20) du liquide ayant traversé la membrane est souple, en ce que cet appareillage comprend de plus des moyens pour mettre le récipient (20) sensiblement à la même pression que celle du récipient souple (17) lors de la phase de retour, et en ce qu'il comprend des moyens pour que la pression d'entrée du compartiment amont (3) de l'appareil à membrane (2), au cours de chaque phase de retour, soit augmentée de la valeur de la pression appliquée sur le récipient souple (17), de façon à conserver sensiblement la même pression transmembranaire dans l'appareil (2) au cours de chaque phase de prélèvement et de chaque phase de retour, la pompe (7) tournant dans le même sens au cours de ces deux phases distinctes.

8. Appareillage selon l'une quelconque des revendications précédentes, caractérisé en ce qu'il comprend une unité logique (22) de commande et de contrôle à laquelle sont reliés notamment les circuits électriques et électroniques.

9. Appareillage selon l'une quelconque des revendications précédentes, caractérisé en ce qu'il comprend un clavier (28) sur lequel on peut choisir le volume de liquide que l'on veut faire arriver dans le récipient (17) lors de chaque phase de prélèvement et sur lequel on peut choisir le volume de liquide que l'on veut collecter dans le récipient (20) lors d'une séance complète.

10. Appareillage selon l'une quelconque des revendications précédentes, caractérisé en ce qu'il comprend une console sur laquelle sont regroupés les éléments constitutifs de l'appareillage.


**Patentansprüche**

1. Anlage zur Verwendung im medizinischen Bereich, dadurch gekennzeichnet, daß sie folgendes enthält:
- eine Vorrichtung (1) zur Entnahme einer Flüssigkeit von einer Person,
- ein Gerät (2) mit Membran, das diese Flüssigkeit in einen Anteil trennt, der die Membran durchquert hat, und in einen Anteil trennt, der die Membran nicht durchquert hat,
- eine erste Leitung (5), die die Entnahmevorrichtung (1) mit dem Einlaß (6) des stromauf gelegenen Abteils (3) des Geräts (2) mit Membran verbindet,
- eine an dieser Leitung (5) gelegene Pumpe (7),
- einen Druckgeber (14) für die in der Leitung (5) zirkulierende Flüssigkeit, wobei dieser Geber zwischen der Pumpe (7) und dem Einlaß (6) des stromauf gelgenen Abteils (3) angeordnet ist,
- eine zweite Leitung (16), die den Auslaß (15) des stromauf gelegenen Abteils (3) des Geräts (2) mit Membran mit einem nachgiebigen Gefäß (17) verbindet, das den Anteil der Flüssigkeit sammelt, die die Membran nicht durchquert hat,
- ein Gefäß (20) zum Sammeln der Flüssigkeit, die die Membran durchquert hat, wobei dieses Gefäß (20) mit dem Auslaß des stromab gelegenen Abteils (4) des Geräts (2) mit Membran in Verbindung steht,
- Mittel zum Feststellen des Flüssigkeitsvolumens, das der Person während jeder Entnahmephase entnommen wurde,
- eine aufblasbare nachgiebige Tasche (18), die im aufgeblasenen Zustand auf das nachgiebige Gefäß (17) einwirkt und während der sogenannten Rücklaufphase den Rücklauf der in dieses Gefäß (17) enthaltenen Flüssigkeit zur Person gestattet, wobei die durch eine einfache Blutentnahmenadel gebildete Vorrichtung dann zur Wiedereinspritzung dieses Anteils, der die Membran nicht durchquert hat, in die Person dient,
- Mittel, die gewährleisten, daß die biegsame Tasche (18) während der sogenannten Rücklaufphase nicht über einen gewissen Sollwert hinaus aufgeblasen wird,
- Mittel zum Aufblasen oder Entlüften der Tasche.

2. Anlage nach Anspruch 1, dadurch gekennzeichnet, daß sie folgendes enthält:
- eine aufblasbare Adernpresse (21),
- Mittel (23, 24, 25), die das Aufblasen der Adernpresse (21) während der Entnahmephase und das Entlüften während der Rücklaufphase gestatten.

3. Anlage nach einem der vorhergehenden Ansprüche, dadurch gekennzeichnet, daß sie eine Vorrichtung (8) enthält, die während der Entnahmephase in Nähe der Entnahmevorrichtung (1) ein Antikoagulationsmittel in die Leitung (5) einführt.

4. Anlage nach einem der vorhergehenden Ansprüche, dadurch gekennzeichnet, daß sie ferner folgendes enthält: Mittel (30), die in jedem Augenblick die im Gefäß (20) enthaltene Flüssigkeitsmenge feststellen, und Mittel für den Übergang von einer Entnahmephase zu einer Rücklaufphase und umgekehrt, bis das gewünschte Flüssigkeitsvolumen im Gefäß (20) erhalten ist, vorzugsweise am Ende einer Rücklaufphase.

5. Anlage nach einem der vorhergehenden Ansprüche, dadurch gekennzeichnet, daß sie ferner einen Blasenfühler (20) und einen Druckgeber (13) zwischen der Entnahmevorrichtung (1) und der Pumpe (7) aufweist.

6. Anlage nach einem der Ansprüche 4 bis 5, dadurch gekennzeichnet, daß während jeder Entnahmephase der auf die ihm zugeordnete Pumpe (7) einwirkende Druckgeber (13) gewährleistet, daß der Druck der Flüssigkeit in dem Teil der Leitung (5) zwischen der Entnahmevorrichtung (1) und der Pumpe (7) nicht unter einen gegebenen minimalen Relativdruck, genannt Schwelldruck, abfällt, daß der auf die ihm zugeordnete Pumpe (7) einwirkend Geber (7) gewährleistet, daß der Einlaßdruck der Flüssigkeit, die in das stromauf gelegene Abteil des Geräts (2) mit Membran eintritt, möglichst nahe einem maximalen Sollwert ist, ohne ihn zu überschreiten, während das nachgiebige Gefäß (17) und das Gefäß (20) sich im wesentlichen auf Atomsphärendruck befinden, daß bei jeder Rücklaufphase die im nachgiebigen Gefäß (17) enthaltene Flüssigkeit durch Aufblasen der Tasche (18) auf einen gegebenen Druck unter Druck gesetzt wird, während die Pumpe (7) im entgegengesetzten Sinn rotiert, damit diese Flüssigkeit zur Entnahmevorrichtung (1) zurückkehrt und dabei durch die Leitung (16) in das stromauf gelegene Abteil (3) des Geräts (2) mit Membran strömt und in das Abteil mit dem gewünschten gegebenen Druck eintritt, wobei der Druck der Flüssigkeit im Gefäß (20) sich auf Atmosphärendruck befindet, und daß der Druckgeber (13) auf die ihm zugeordnete Pumpe einwirkt, damit diese zur Person zurückkehrende Flüssigkeit keinen Druck hat, der höher als ein Sollwert ist, und sich möglichst nahe diesem Druckwert hält, daß der Druckgeber (14) auf die ihm zugeordnete Pumpe (7) einwirkt, damit der Auslaßdruck des Geräts (2) mit Membran dem Atomsphärendruck möglichst nahe ist, wobei der Geber (14) gegebenenfalls wirkt, um den Druck der aufblasbaren Tasche (18) und somit der im Gefäß (17) enthaltenen Flüssigkeit abzusenken.

7. Anlage nach einem der Ansprüche 1 bis 5, dadurch gekennzeichnet, daß sie ferner eine Leitung (16b) enthält, die sich vom nachgiebigen Gefäß (17) zur Leitung (5) erstreckt, an die sie vorzugsweise zwischen der Blasenfalle (12) und der Pumpe (7) angeschlossen ist, daß das Gefäß (20) für die Flüssigkeit, die die Membran durchquert hat, biegsam ist, daß diese Anlage ferner Mittel enthält, die während der Rücklaufphase das Gefäß (20) im wesentlichen auf denselben Druck wie denjenigen des biegsamen Gefäßes (17) bringen, und daß sie Mittel enthält, damit während jeder Rücklaufphase der Einlaßdruck des stromauf gelegenen Abteils (3) des Geräts (2) mit Membran um den auf das biegsame Gefäß (7) gegebenen Druckwert erhöht wird, und zwar derart, daß während jeder Entnahmephase und jeder Rücklaufphase derselbe Transmembrandruck im Gerät (2) im wesentlichen beibehalten wird, wobei die Pumpe (7) während dieser beiden gesonderten Phasen im gleichen Sinn rotiert.

8. Anlage nach einem der vorhergehenden Ansprüche, dadurch gekennzeichnet, daß sie eine logische Steuer- und Kontrolleiheit (22) enthält, mit der insbesondere die elektrischen Kreise verbunden sind.

9. Anlage nach einem der vorhergehenden Ansprüche, dadurch gekennzeichnet, daß sie eine Tastatur (28) enthält, auf der man das Flüssigkeitsvolumen wählen kann, das während jeder Entnahmephase in das Gefäß (17) gelangen soll, und auf der man das Flüssigkeitsvolumen wählen kann, das während einer vollständigen Sitzung im Gefäß (20) gesammelt werden soll.

10. Anlage nach einem der vorhergehenden Ansprüche, dadurch gekennzeichnet, daß sie eine Konsole enthält, auf der die die Anlage bildenden Elemente gruppiert sind.

## Claims

1. Installation which can be used in the medical field, characterized in that it comprises:
   a device (1) for the withdrawal of a liquid from a subject,
   an apparatus (2) containing a membrane which separates this liquid into a fraction which has passed through the membrane and a fraction which has not passed through the membrane,
   a first pipeline (5) connecting the withdrawal device (1) to the inlet (6) of the upstream compartment (3) of the membrane-containing apparatus (2),
   a pump (7) arranged on this pipeline (5),
   a pressure sensor (14) of the liquid circulating in the pipeline (5), the said sensor being located between the pump (7) and the inlet (6) of the upstream compartment (3),
   a second pipeline (16) connecting the outlet (15) of the upstream compartment (3) of the membrane-containing apparatus (2) to a flexible container (17) for collecting the fraction of the liquid which has not passed through the membrane,
   a container (20) for collecting the liquid which has passed through the membrane, this container (20) being in communication with the outlet of the downstream compartment (4) of the membrane-containing apparatus (2),
   means for knowing the volume of liquid withdrawn from the subject during each withdrawal phase,
   an inflatable flexible bag (18) acting, when inflated, on the flexible container (17) and enabling the liquid contained in this container (17) to be returned to the subject during the phase referred to as the return phase, the device (1) consisting of a single blood sampling needle which serves, in this case, for reinjecting this fraction which has not passed through the membrane to the subject,
   means for ensuring that the flexible bag (18) is not inflated beyond a certain predetermined pressure during the phase referred to as the return phase,
   means (23, 19) for inflating and deflating the said bag.

2. Installation according to claim 1, characterized in that it additionally comprises:
   an inflatable tourniquet (21),
   means (23, 24, 25) which enable the tourniquet (21) to be inflated during the withdrawal phase and deflated during the return phase.

3. Installation according to any one of the preceding claims, characterized in that it comprises a device (8) for introducing, during the withdrawal phase, an anticoagulant into the pipeline (5), close to the withdrawal device (1).

4. Installation according to any one of the preceding claims, characterized in that it additionally comprises means (30) for knowing at any time the quantity of liquid contained in the container (20) and means for passing from a withdrawal phase to a return phase and vice versa, until the desired volume of liquid is obtained in the container (20), preferably at the end of a return phase.

5. Installation according to any one of the preceding claims, characterized in that it additionally comprises a bubble detector (12) and a pressure sensor (13) between the withdrawal device (1) and the pump (7).

6. Installation according to either of claims 4 and 5, characterized in that during each withdrawal phase, the pressure sensor (13) acting on the pump (7) which is under its control makes it possible to ensure that the pressure of the liquid in the portion of the line (5) located between the withdrawal device (1) and the said pump (7) does not fall below a predetermined minimum relative pressure, called the threshold pressure, in that the sensor (14) acting on the pump (7) which is under its control makes it possible to ensure that the entry pressure of the liquid entering the upstream compartment (3) of the membrane-containing apparatus (2) is as close as possible to a predetermined maximum pressure without exceeding it, whereas the flexible container (17) and the container (20) are substantially at atmospheric pressure, in that at each return phase the liquid contained in the flexible container (17) is pressurized to a predetermined pressure by the inflation of the bag (18) whereas the pump (7) rotates in the opposite direction, so that this liquid returns to the withdrawal device (1) by way of the pipeline (16) in the upstream compartment (3) of the membrane-containing apparatus and enters the said compartment at the desired predetermined pressure, the pressure of the liquid in the container (20) being at atmospheric pressure, and in that the pressure sensor (13) acts on the pump (7) which is under its control so that this liquid returning to the subject is not at a pressure greater than a predetermined value and is kept as close as possible to this pressure value, and in that the pressure sensor (14) acts on the pump (7) which is under its control so that the pressure at the outlet of the membrane-containing apparatus (2) is as close as possible to atmospheric pressure, the sensor (14) acting, if required, to lower the pressure in the inflatable bag (18) and thus of the liquid contained in the container (17).

7. Installation according to any one of claims 1 to 5, characterized in that it additionally comprises a pipeline (16b) going from the flexible container (17) to the line (5) with which it is connected, preferably bet ween the bubble trap (12) and the pump (7), in that the container (20) for the liquid which has passed through the membrane is flexible, in that this installation additionally comprises means for pressurizing the container (20) substantially to the same pressure as that of the flexible container (17) during the return phase, and in that it comprises means which ensure that the entry pressure of the upstream compartment (3) of the membrane-containing apparatus (2), during each return phase, is increased by the value of the pressure applied to the flexible container (17), so as to maintain substantially the same pressure across the membrane in the apparatus (2) during each withdrawal phase and each return phase, the pump (7) rotating in the same direction during these two separate phases.

8. Installation according to any one of the preceding claims, characterized in that it includes a logic unit (22) for controlling and monitoring, to which especially the electrical and electronic circuits are connected.

9. Installation according to any one of the preceding claims, characterized in that it comprises a keyboard (28) on which the volume of liquid desired to be brought to the container (17) during each withdrawal phase may be chosen and on which the volume of liquid desired to be collected in the container (20) during a complete session may be chosen.

10. Installation according to any one of the preceding claims, characterized in that it comprises a console on which the constituent components of the installation are gathered.

Fig. 1

0 132 210

Fig. 2

0 132 210

Fig. 3